# EUROPÄISCHE PATENTSCHRIFT

(11) **EP 3 746 286 B1**
(45) Veröffentlichungstag und Bekanntmachung des Hinweises auf die Patenterteilung: **05.01.2022**
(21) Anmeldenummer: 19703966.2
(22) Anmeldetag: 30.01.2019
(51) Int. Cl.: B29C 65/10, B29C 65/12, B29C 65/14, B29C 65/20, B29C 65/42, B29C 65/48, B29C 65/52

(54) **VERFAHREN ZUR HERSTELLUNG EINES BEHÄLTERS UND BEHÄLTER**
METHOD OF MANUFACTURING A CONTAINER AND CONTAINER
PROCÉDÉ DE FABRICATION D'UN RÉCIPIENT ET RÉCIPIENT

(30) Priorität: 30.01.2018 DE 102018201349
(43) Veröffentlichungstag der Anmeldung: 09.12.2020
(73) Patentinhaber: Greiner Bio-One GmbH, 72636 Frickenhausen (DE)
(72) Erfinder: STAEMMLER, Lutz, 72660 Beuren (DE); THUMM, Andreas, 72766 Reutlingen (DE)
(74) Vertreter: Schrell, Andreas
(86) Internationale Anmeldenummer: PCT/EP2019/052295
(87) Internationale Veröffentlichungsnummer: WO 2019/149779

(56) Entgegenhaltungen:
- DE-U1-202009 009 762
- GB-A- 848 967

## Beschreibung

Die vorliegende Erfindung betrifft ein Verfahren zur Herstellung eines zumindest teilweise aus einem thermoplastischen Kunststoff aufgebauten Behälters, umfassend mindestens ein erstes Kompartimentelement und mindestens ein mit dem ersten in einem Verbindungsbereich thermoplastisch verschweißtes zweites Kompartimentelement, sowie einen nach dem erfindungsgemäßen Verfahren herstellbaren, bevorzugt hergestellten, Kunststoffbehälter.

Eine besondere Herausforderung bei der Herstellung von Behältern aus thermoplastischen Kunststoff ist das feste, insbesondere rückstandslose, Verbinden von mehreren den Behälter bildenden Kunststoffelementen. Gegenwärtig kommen zur Verschweißung von Kunststoffteilen im Stand der Technik unterschiedliche Verfahren zum Einsatz.

Das sogenannte Laserschweißen basiert darauf, dass zwei unterschiedliche Kunststoffe verwendet werden, von denen der eine für den Laser transparent ist und der andere die Energie des Lasers absorbiert, so dass es lokal zur Erhitzung des thermoplastischen Kunststoffes kommt und unter Anlegung eines Fügedrucks eine Verschweißung zwischen den einzelnen Kunststoffteilen entsteht.

Beim sogenannten Ultraschallverfahren werden gezielt bestimmte Teile des Kunststoffs mittels Ultraschall erhitzt, so dass unter Fügedruck eine Verbindung der erhitzten thermoplastischen Kunststoffteile stattfindet. Nachteilig bei diesem Verfahren ist insbesondere die Gefahr der Partikelbildung.

Eine weitere Möglichkeit einzelne Kunststoffelemente miteinander zu verbinden besteht darin, die einzelnen Elemente des Kunststoffbehälters miteinander zu verkleben, wodurch nachteiligerweise jedoch die Gefahr besteht, dass Reste des Klebstoffs, beziehungsweise des verwendeten Lösungsmittels, an der Verbindungsstelle verbleiben, wodurch der Einsatzbereich eines auf diese Weise hergestellten Behälters deutlich einschränkt wird.

Beim Heizelement-, Heizgas- oder Rotationsschweißen, werden thermoplastische Kunststoffteile zunächst durch Erwärmen plastifiziert und anschließend durch Anlegen eines Fügedrucks miteinander verbunden. Das Heizelementschweißen umfasst das Kontaktieren mindestens eines der beiden zu verschweißenden thermoplastischen Kunststoffteile mit einem Heizelement, wodurch die Fügefläche lokal plastifiziert wird. Anschließend erfolgt in einem zweiten Schritt das Fügen der zu verschweißenden Kunststoffteile durch Anlegen eines Fügedrucks. Im Unterschied zu Heizelementschweißen wird beim Heizgasschweißen die Fügefläche mindestens eines der zu verschweißenden Kunststoffteile mittels eines Heizgasstroms lokal plastifiziert. Auch bei diesem Verfahren erfolgt das Verbinden der Kunststoffteile durch das Anlegen eines Fügedrucks. Beim Rotationsschweißen erfolgt das Plastifizieren des Kunststoffs durch Reibungswärme, die beim Rotieren des aufzuschweißenden Kunststoffteils auf einem verdrehsicher arretierten zweiten Kunststoffteil erzeugt wird. Im Bereich der sich kontaktierenden Fügeflächen kommt es so zum Anschmelzen der zu verbindenden Kunststoffteile, die durch den angelegten Druck fest miteinander verbunden werden.

Die DE 3609775 A1 betrifft eine Vorrichtung und die GB 848 967 A betrifft ein Verfahren zum materialschlüssigen Verbinden von mindestens im Bereich der Verbindungsstellen thermoplastischem Kunststoff. Die Vorrichtung umfasst ein Wärme- und/oder Mikrowellenstrahlorgan und eine Zuführdüse, über die im Kontaktbereich der zwei Kunststoffteile plastifizierter Kunststoff zugeführt wird, wodurch die, zumindest im Bereich der Verbindungsstellen thermoplastischen, Kunststoffteile materialschlüssig miteinander verbunden werden.

Die DE 20 2009 009762 U1 betrifft ein Behältnis, umfassend zwei Kompartimentelemente, die mittels Ultraschallschweißverfahren, Laserschweißen, Heißprägeverfahren oder Klebeverfahren miteinander verbunden werden können. Insbesondere offenbart die DE 20 2009 009762 U1 ein Verfahren, das die Schritte a), b) und d) des Anspruchs 1 umfasst.

Aufgabe der vorliegenden Verbindung ist es, ein Verfahren zur Herstellung eines mindestens teilweise aus einem thermoplastischen Kunststoff aufgebauten Behälters bereitzustellen, bei welchem die Nachteile der im Stand der Technik bekannten Fügeverfahren überwunden werden, so dass es vorteilhafterweise ohne die Notwendigkeit des Anlegens eines Fügedrucks zum insbesondere Klebstoff-, Lösungsmittel- und Partikel-freien Verschweißen der einzelnen den Behälter bildenden Kunststoffteile kommt.

Die vorliegende Erfindung löst das ihr zugrundeliegende technische Problem insbesondere durch den Gegenstand der unabhängigen Ansprüche.

Die Erfindung betrifft insbesondere ein Verfahren zur Herstellung eines zumindest teilweise aus einem thermoplastischen Kunststoff aufgebauten Behälters, umfassend mindestens ein erstes Kompartimentelement und mindestens ein mit dem ersten in einem Verbindungsbereich thermoplastisch verschweißtes zweites Kompartimentelement, wobei das Verfahren folgende Verfahrensschritte umfasst:
a) Bereitstellen mindestens eines ersten und eines zweiten Kompartimentelements, welche jeweils ein Plattenelement mit mindestens einer die Außenkanten des Kompartimentelements umfassenden Verbindungszone aus einem thermoplastischen Material aufweisen, wobei zumindest das zweite Kompartimentelement eine um den Außenumfang des Plattenelements angeordnete Seitenwand mit jeweils einer distalen ersten und proximalen zweiten Außenkante umfasst,
b) Aneinanderfügen der mindestens zwei Kompartimentelemente, so dass sich die Verbindungszonen aus thermoplastischem Kunststoff kontaktieren und eine Außenkante des ersten Kompartimentelements vollumfänglich an die erste Außenkante des zweiten Kompartimentelements stößt und ein vollumfänglicher Stumpfstoß zwischen zwei Kompartimentelementen gebildet wird,
c) Verschweißen der mindestens zwei Kompartimentelemente mittels mindestens einer temperaturregulierten Düse, wobei die mindestens eine Düse den Stumpfstoß kontaktierend um den Umfang des Stumpfstoßes geführt wird oder der Stumpfstoß die mindestens eine Düse kontaktierend an der mindestens einen Düse entlanggeführt wird, wobei dabei die jeweilige erste und zweite Außenkante der Kompartimentelemente plastifiziert wird und gleichzeitig plastifizierter Schweißzusatz auf den Stumpfstoß übertragen wird, so dass eine Schweißnaht zwischen den beiden Kompartimentelementen gebildet wird, und
d) Erhalt eines Kunststoffbehälters, wobei die mindestens zwei Kompartimentelemente miteinander verschweißt sind.

In einer bevorzugten Ausführungsform der vorliegenden Erfindung ist das erste Kompartimentelement ein Plattenelement. Gemäß dieser Ausführungsform umfasst das zweite Kompartimentelement ein Plattenelement und eine um den Außenumfang des Plattenelements angeordnete Seitenwand mit jeweils einer parallel zum Plattenelement angeordneten distalen ersten und proximalen zweiten Außenkante.

In einer weiteren bevorzugten Ausführungsform der vorliegenden Erfindung umfasst das erste und/oder zweite Kompartimentelement eine um den Außenumfang des Plattenelements angeordnete Seitenwand mit jeweils einer parallel zum Plattenelement angeordneten distalen ersten und proximalen zweiten Außenkante. Bevorzugt ist die Seitenwand des ersten und/oder zweiten Kompartimentelements orthogonal zur vom Plattenelement gebildeten Ebene um den Außenumfang des Plattenelements angeordnet. Bevorzugt verläuft die vom Plattenelement gebildete Ebene parallel zu den von der distalen ersten und der proximalen zweiten Außenkante gebildeten Ebenen. Bevorzugt ist der Abstand der vom Plattenelement gebildeten Ebene zur von der distalen ersten Außenkante gebildeten Ebene größer als der Abstand der vom Plattenelement gebildeten Ebene zur von der proximalen zweiten Außenkante gebildeten Ebene. Bevorzugt ist der Abstand der vom Plattenelement gebildeten Ebene zur von der distalen ersten Außenkante gebildeten Ebene kleiner als der Abstand der vom Plattenelement gebildeten Ebene zur von der proximalen zweiten Außenkante gebildeten Ebene. Bevorzugt ist der Abstand der vom Plattenelement gebildeten Ebene zur von der distalen ersten Außenkante gebildeten Ebene identisch mit dem Abstand der vom Plattenelement gebildeten Ebene zur von der proximalen zweiten Außenkante gebildeten Ebene.

In einer bevorzugten Ausführungsform der vorliegenden Erfindung besitzt die Seitenwand eines Kompartimentelements eine Höhe von 2 bis 200 mm, bevorzugt 2 bis 180 mm, bevorzugt 2 bis 160 mm, bevorzugt 2 bis 140 mm, bevorzugt 2 bis 120 mm, bevorzugt 2 bis 100 mm, bevorzugt 2 bis 80 mm, bevorzugt 2 bis 60 mm, bevorzugt 4 bis 40 mm, bevorzugt 4 bis 30 mm, bevorzugt 4 bis 25 mm, bevorzugt 4 bis 20 mm, bevorzugt 4 bis 18 mm, bevorzugt 4 bis 16 mm, bevorzugt 6 bis 14 mm, bevorzugt 6 bis 12 mm, bevorzugt 8 bis 12 mm, bevorzugt 10 mm. Bevorzugt beträgt die Höhe der Seitenwand eines Kompartimentelements mindestens 2 mm, bevorzugt mindestens 4 mm, bevorzugt mindestens 6 mm, bevorzugt mindestens 8 mm, bevorzugt mindestens 10 mm.

In einer weiteren bevorzugten Ausführungsform der vorliegenden Erfindung besitzen die Seitenwände der einzelnen Kompartimentelemente unterschiedliche Höhen. Bevorzugt besitzen alle Seitenwände der einzelnen Kompartimentelemente die gleiche Höhe.

In einer bevorzugten Ausführungsform der vorliegenden Erfindung besitzt die Seitenwand eines Kompartimentelements eine Dicke von 0,1 bis 10 mm, bevorzugt 0,1 bis 9 mm, bevorzugt 0,2 bis 8 mm, bevorzugt 0,3 bis 7 mm, bevorzugt 0,4 bis 6 mm, bevorzugt 0,5 bis 5 mm, bevorzugt 0,6 bis 4,5 mm, bevorzugt 0,7 bis 4 mm, bevorzugt 0,8 bis 3,5 mm, bevorzugt 1 bis 3 mm, bevorzugt 1,25 bis 2,5 mm, bevorzugt 1,5 bis 2,5 mm, bevorzugt 1,75 bis 2,25 mm, bevorzugt 2 mm. Bevorzugt beträgt die Dicke der Seitenwand eines Kompartimentelements mindestens 0,1 mm, bevorzugt mindestens 0,2 mm, bevorzugt mindestens 0,3 mm, bevorzugt mindestens 0,4 mm, bevorzugt mindestens 0,5 mm, bevorzugt mindestens 0,6 mm, bevorzugt mindestens 0,7 mm, bevorzugt mindestens 0,8 mm, bevorzugt mindestens 0,9 mm, bevorzugt mindestens 1 mm.

In einer weiteren bevorzugten Ausführungsform der vorliegenden Erfindung besitzen die Seitenwände der einzelnen Kompartimentelemente unterschiedliche Dicken. Bevorzugt besitzen alle Seitenwände der einzelnen Kompartimentelemente die gleiche Dicke.

In einer bevorzugten Ausführungsform der vorliegenden Erfindung ist das Plattenelement der mindestens zwei Kompartimentelemente quadratisch, rechteckig, oval oder rund. Bevorzugt ist das Plattenelement der mindestens zwei Kompartimentelemente quadratisch. Bevorzugt ist das Plattenelement der mindestens zwei Kompartimentelemente rechteckig. Bevorzugt ist das Plattenelement der mindestens zwei Kompartimentelemente oval. Bevorzugt ist das Plattenelement der mindestens zwei Kompartimentelemente rund.

In einer bevorzugten Ausführungsform der vorliegenden Erfindung wird in Verfahrensschritt a) mindestens ein weiteres Kompartimentelement bereitgestellt, in Verfahrensschritt b) das weitere Kompartimentelement mit seiner ersten Außenkante vollumfänglich an die zweite Außenkante des zweiten oder eines gegebenfalls vorliegenden weiteren Kompartimentelement angefügt und in Verfahrensschritt c) der zwischen den aneinandergefügten Kompartimentelementen entstandenen Stumpfstoß verschweißt.

In einer bevorzugten Ausführungsform der vorliegenden Erfindung umfasst jedes weitere Kompartimentelement ein Plattenelement und eine um den Außenumfang des Plattenelements angeordnete Seitenwand mit jeweils einer parallel zum Plattenelement angeordneten distalen ersten und proximalen zweiten Außenkante.

In einer bevorzugten Ausführungsform der vorliegenden Erfindung werden in Verfahrensschritt b) die Kompartimentelemente gegeneinander drehsicher aneinandergefügt.

In einer bevorzugten Ausführungsform der vorliegenden Erfindung bestehen mindestens ein Kompartimentelement und der Schweißzusatz aus den gleichen Materialien, bevorzugt aus dem gleichen Material.

In einer bevorzugten Ausführungsform der vorliegenden Erfindung wird der Schweißzusatz von der mindestens einen temperaturregulierten Düse plastifiziert. Bevorzugt wird der Schweißzusatz in der mindestens einen temperaturregulierten Düse plastifiziert.

In einer bevorzugten Ausführungsform der vorliegenden Erfindung beträgt die Temperatur der mindestens einen temperaturregulierten Düse 150 bis 320 °C, bevorzugt 160 bis 310 °C, bevorzugt 170 bis 300 °C, bevorzugt 180 bis 290 °C, bevorzugt 190 bis 280 °C, bevorzugt 200 bis 275 °C, bevorzugt 210 bis 270 °C, bevorzugt 220 bis 265 °C, bevorzugt 225 bis 260 °C, bevorzugt 230 bis 255 °C, bevorzugt 235 bis 250 °C, bevorzugt 240 bis 250 °C. Bevorzugt beträgt die Temperatur der mindestens einen temperaturregulierten Düse mindestens 150 °C, bevorzugt mindestens 160 °C, bevorzugt mindestens 170 °C, bevorzugt mindestens 180 °C, bevorzugt mindestens 190 °C, bevorzugt mindestens 200 °C, bevorzugt mindestens 210 °C, bevorzugt mindestens 220 °C, bevorzugt mindestens 230 °C, bevorzugt mindestens 240 °C, bevorzugt mindestens 250 °C. Dabei hängt die eingestellte Temperatur der mindestens einen temperaturregulierten Düse von der spezifischen Schmelztemperatur des im erfindungsgemäßen Verfahren gewählten mindestens einen thermoplastischen Materials ab, und wird so gewählt, dass es durch Kontaktieren der mindestens einen temperaturregulierten Düse mit dem zwischen den mindestens zwei Kompartimentelementen gebildeten Stumpfstoß zur Plastifizierung der jeweiligen ersten und zweiten Außenkante im Bereich der Verbindungszonen der Kompartimentelemente kommt und gleichzeitig plastifizierter Schweißzusatz durch die mindestens eine temperaturregulierte Düse auf den Stumpfstoß übertragen werden kann.

In einer bevorzugten Ausführungsform der vorliegenden Erfindung handelt es sich bei dem mindestens einen thermoplastischen Material um Polystyrol und die Temperatur der mindestens einen temperaturregulierten Düse beträgt 225 bis 240 °C, bevorzugt 230 bis 235 °C. In einer weiteren bevorzugten Ausführungsform handelt es sich bei dem mindestens einen thermoplastischen Material um Polyurethan und die Temperatur der mindestens einen temperaturregulierten Düse beträgt 200 bis 240 °C, bevorzugt 205 bis 235 °C, bevorzugt 220 °C. In einer weiteren bevorzugten Ausführungsform handelt es sich bei dem mindestens einen thermoplastischen Material um Polyethylenterephthalat und die Temperatur der mindestens einen temperaturregulierten Düse beträgt 205 bis 255 °C, bevorzugt 210 bis 250 °C, bevorzugt 230 °C. In einer weiteren bevorzugten Ausführungsform handelt es sich bei dem mindestens einen thermoplastischen Material um Acrylnitril-Butadien-Styrol und die Temperatur der mindestens einen temperaturregulierten Düse beträgt 235 bis 265 °C, bevorzugt 240 bis 260 °C, bevorzugt 250 °C. In einer weiteren bevorzugten Ausführungsform handelt es sich bei dem mindestens einen thermoplastischen Material um Polyamid und die Temperatur der mindestens einen temperaturregulierten Düse beträgt 235 bis 265 °C, bevorzugt 240 bis 260 °C, bevorzugt 250 °C. In einer weiteren bevorzugten Ausführungsform handelt es sich bei dem mindestens einen thermoplastischen Material um Polypropylen und die Temperatur der mindestens einen temperaturregulierten Düse beträgt 245 bis 275 °C, bevorzugt 250 bis 270 °C, bevorzugt 260 °C. In einer weiteren bevorzugten Ausführungsform handelt es sich bei dem mindestens einen thermoplastischen Material um Polyethylen (LDPE) und die Temperatur der mindestens einen temperaturregulierten Düse beträgt 155 bis 265 °C, bevorzugt 160 bis 260 °C, bevorzugt 180 bis 240 °C, bevorzugt 200 bis 220 °C, bevorzugt 210 °C. In einer weiteren bevorzugten Ausführungsform handelt es sich bei dem mindestens einen thermoplastischen Material um Polyethylen (HDPE) und die Temperatur der mindestens einen temperaturregulierten Düse beträgt 255 bis 305 °C, bevorzugt 260 bis 300 °C, bevorzugt 280 °C.

In einer bevorzugten Ausführungsform der vorliegenden Erfindung bestehen die Verbindungszonen der mindestens zwei Kompartimentelemente aus einem Material umfassend ein oder mehrere Thermoplasten ausgewählt aus der Gruppe bestehend aus Polystyrol, Polypropylen, Polycarbonat, Polyethylenterephtalat und Polyethylen.

In einer weiteren bevorzugten Ausführungsform besteht mindestens eines der mindestens zwei Kompartimentelemente aus einem Material umfassend ein oder mehrere Thermoplasten ausgewählt aus der Gruppe bestehend aus Polystyrol, Polypropylen, Polycarbonat, Polyethylenterephtalat und Polyethylen.

In einer bevorzugten Ausführungsform der vorliegenden Erfindung ist das Material des Schweißzusatzes ein oder mehrere Thermoplasten ausgewählt aus der Gruppe bestehend aus Polystyrol, Polypropylen, Polycarbonat, Polyethylenterephthalat und Polyethylen.

In einer weiteren bevorzugten Ausführungsform der vorliegenden Erfindung bestehen mindestens eines der mindestens zwei Kompartimentelement und der Schweißzusatz aus dem gleichen Material. Bevorzugt bestehen mindestens eines der mindestens zwei Kompartimentelement und der Schweißzusatz aus einem oder mehreren Thermoplasten ausgewählt aus der Gruppe bestehend aus Polystyrol, Polypropylen, Polycarbonat, Polyethylenterephtalat und Polyethylen.

In einer weiteren bevorzugten Ausführungsform der vorliegenden Erfindung wird der Schweißzusatz in Form von Granulat oder eines kontinuierlichen Strangs der mindestens einen Düse zugeführt. Bevorzugt wird der Schweißzusatz in Form von Granulat der mindestens einen Düse zugeführt. Bevorzugt wird der Schweißzusatz in Form eines kontinuierlichen Strangs, bevorzugt in Form eines Schweißdrahts, der mindestens einen Düse zugeführt.

In einer bevorzugten Ausführungsform der vorliegenden Erfindung werden in einem weiteren Verfahrensschritt b') die Kompartimentelemente im Bereich des Stumpfstoßes vorgewärmt. Bevorzugt erfolgt in Verfahrensschritt b') die Vorwärmung der Kompartimentelemente im Bereich des Stumpfstoßes durch Heißluft, Infrarot- und/oder Mikrowellenstrahlung.

In einer bevorzugten Ausführungsform der vorliegenden Erfindung wird in einem weiteren Verfahrensschritt c') die Schweißnaht erwärmt. Bevorzugt wird in Verfahrensschritt c') die Schweißnaht durch Heißluft, Infrarot- und/oder Mikrowellenstrahlung erwärmt.

In einer bevorzugten Ausführungsform der vorliegenden Erfindung wird durch das Aufeinanderstoßen der Kompartimentelemente in Verfahrensschritt b) mindestens eine Vertiefung im Bereich des Stumpfstoßes zwischen den Kompartimentelementen gebildet.

In einer bevorzugten Ausführungsform der vorliegenden Erfindung erfolgt das Verschweißen der mindestens zwei Kompartimentelemente mittels mindestens einer temperaturregulierten Düse in Verfahrensschritt c) dadurch, dass die mindestens eine Düse den Stumpfstoß kontaktierend, um den Umfang des Stumpfstoßes geführt wird und gleichzeitig plastifizierter Schweißzusatz auf den Stumpfstoß übertragen wird, so dass eine Schweißnaht zwischen den beiden Kompartimentelementen gebildet wird.

In einer weiteren bevorzugten Ausführungsform erfolgt das Verschweißen der mindestens zwei Kompartimentelemente mittels mindestens einer temperaturregulierten Düse in Verfahrensschritt c) dadurch, dass der Stumpfstoß die mindestens eine Düse kontaktierend an der mindestens einen Düse entlanggeführt wird und gleichzeitig plastifizierter Schweißzusatz auf den Stumpfstoß übertragen wird, so dass eine Schweißnaht zwischen den beiden Kompartimentelementen gebildet wird.

Bevorzugt erfolgt das Verschweißen der mindestens zwei Kompartimentelemente mittels mindestens einer temperaturregulierten Düse in Verfahrensschritt c) dadurch, dass der Stumpfstoß und die mindestens einen Düse einander kontaktierend relativ zueinander bewegt werden und gleichzeitig plastifizierter Schweißzusatz auf den Stumpfstoß übertragen wird, so dass eine Schweißnaht zwischen den beiden Kompartimentelementen gebildet wird.

In einer weiteren bevorzugten Ausführungsform der vorliegenden Erfindung hat die mindestens eine temperaturregulierte Düse einen Durchmesser von 0,3 bis 2 mm, bevorzugt 0,3 bis 1,8 mm, bevorzugt 0,4 bis 1,6 mm, bevorzugt 0,4 bis 1,4 mm, bevorzugt 0,5 bis 1,2 mm, bevorzugt 0,5 bis 1 mm, bevorzugt 0,5 bis 0,8 mm. Die Verwendung mindestens einer temperaturregulierten Düse mit einem geringen Düsendurchmesser ermöglich es vorteilhafterweise, dass die in Schritt c) zwischen den Kompartimentelementen gebildete Schweißnaht eine besonders geringe Breite aufweist.

In einer bevorzugten Ausführungsform der vorliegenden Erfindung liegt die Breite der in Schritt c) zwischen den Kompartimentelementen gebildeten Schweißnaht bevorzugt unter 5 mm, bevorzugt unter 4,5 mm, bevorzugt unter 4 mm, bevorzugt unter 3,5 mm, bevorzugt unter 3 mm, bevorzugt unter 2,5 mm, bevorzugt unter 2 mm.

In einer bevorzugten Ausführungsform der vorliegenden Erfindung werden in Verfahrensschritt c) mindestens drei, bevorzugt mindestens vier, bevorzugt mindestens fünf, bevorzugt mindestens sechs, bevorzugt alle, in Verfahrensschritt a) bereitgestellten und in Verfahrensschritt b) aneinandergefügten Kompartimentelemente gleichzeitig miteinander verschweißt. Bevorzugt werden in Verfahrensschritt c) mindestens drei, bevorzugt mindestens vier, bevorzugt mindestens fünf, bevorzugt mindestens sechs, bevorzugt alle, in Verfahrensschritt a) bereitgestellten und in Verfahrensschritt b) aneinandergefügten Kompartimentelemente nacheinander verschweißt.

In einer bevorzugten Ausführungsform der vorliegenden Erfindung wird die mindestens eine temperaturregulierte Düse in Verfahrensschritt c) vollständig oder partiell in die mindestens eine Vertiefung eingeführt. Bevorzugt wird in Verfahrensschritt c) die mindestens eine temperaturregulierte Düse vollständig in die mindestens eine Vertiefung eingeführt. Bevorzugt wird in Verfahrensschritt c) die mindestens eine temperaturregulierte Düse partiell in die mindestens eine Vertiefung eingeführt. In dieser Ausführungsform kontaktiert die mindestens eine temperaturregulierte Düse beim vollständigen oder partiellen Einführen in die mindestens eine Vertiefung die beiden die Vertiefung bildenden Kompartimentelemente, insbesondere die beiden Außenkanten der die Vertiefung bildenden Kompartimentelemente. Durch das Kontaktieren kommt es im Verbindungsbereich zum Anschmelzen des thermoplastischen Materials der Kompartimentelemente, wobei die Vertiefung zumindest teilweise, bevorzugt vollständig, durch die mindestens eine temperaturregulierte Düse mit dem Schweißzusatz ausgefüllt wird, wodurch es zum insbesondere Klebstoff-, Lösungsmittel- und Partikel-freien Verschweißen der Kompartimentelemente kommt.

In einer bevorzugten Ausführungsform der vorliegenden Erfindung sind die Kompartimentelemente für die Kultivierung und/oder Untersuchung von Zellen geeignet. Bevorzugt ist mindestens ein Kompartimentelement für die Kultivierung und/oder Untersuchung von Zellen geeignet. Bevorzugt ist mindestens ein Kompartimentelement eine Zellkulturschale.

In einer bevorzugten Ausführungsform der vorliegenden Erfindung umfasst der in Schritt d) erhaltene Kunststoffbehälters mindestens zwei, bevorzugt mindestens drei, bevorzugt mindestens vier, bevorzugt mindestens fünf, bevorzugt mindestens sechs, bevorzugt mindestens sieben, bevorzugt mindestens acht, bevorzugt mindestens neun, bevorzugt mindestens zehn, bevorzugt mindestens 20, bevorzugt mindestens 30, bevorzugt mindestens 40, bevorzugt mindestens 50, bevorzugt mindestens 100, bevorzugt mindestens 200, Kompartimentelemente, die miteinander verschweißt sind. Bevorzugt umfasst der in Schritt d) erhaltene Kunststoffbehälters 2 bis 20, bevorzugt 2 bis 18, bevorzugt 2 bis 16, bevorzugt 2 bis 14, bevorzugt 2 bis 12, bevorzugt 2 bis 10, bevorzugt 4 bis 10, bevorzugt 4 bis 8, bevorzugt 4 bis 6, Kompartimentelemente, die miteinander verschweißt sind.

In einer bevorzugten Ausführungsform der vorliegenden Erfindung weist das mindestens eine Kompartimentelement einen verbindenden, bevorzugt zentralen, Kanal auf. Bevorzugt weist das mindestens eine Kompartimentelement einen verbindenden, bevorzugt zentralen, Kanal als Versorgungskanal für Zellkulturmedium auf. Bevorzugt weist jedes der Kompartimentelemente einen verbindenden, bevorzugt zentralen, Kanal auf. Bevorzugt weist jedes der Kompartimentelemente einen verbindenden, bevorzugt zentralen, Kanal als Versorgungskanal für Zellkulturmedium auf.

In einer bevorzugten Ausführungsform der vorliegenden Erfindung weist mindestens ein Kompartimentelement einen Entsorgungskanal auf. Bevorzugt weist mindestens ein Kompartimentelement einen Entsorgungskanal für verbrauchtes Zellkulturmedium auf.

In einer bevorzugten Ausführungsform der vorliegenden Erfindung ist ein Stoffaustausch, bevorzugt Gas- und/oder Flüssigkeitsaustausch, zwischen einzelnen Kompartimentelementen möglich. Bevorzugt ist ein Stoffaustausch, bevorzugt Gas- und/oder Flüssigkeitsaustausch, zwischen allen Kompartimentelementen möglich.

In einer bevorzugten Ausführungsform der vorliegenden Erfindung weist mindestens ein, bevorzugt genau ein, Kompartimentelement mindestens eine verschließbare, bevorzugt wiederverschließbare, Öffnung auf. In einer bevorzugten Ausführungsform weist die mindestens eine Öffnung ein Gewinde, bevorzugt ein Außengewinde, auf. Bevorzugt kann der zumindest teilweise aus thermoplastischem Kunststoff aufgebaute Behälter über die mindestens eine Öffnung gefüllt und entleert werden.

Die vorliegende Erfindung betrifft auch einen Kunststoffbehälter umfassend mindestens zwei Kompartimentelemente herstellbar, bevorzugt hergestellt, gemäß dem erfindungsgemäßen Verfahren.

In einer bevorzugten Ausführungsform ist der Kunststoffbehälter umfassend mindestens zwei Kompartimentelemente ein Zellkulturgefäß. Bevorzugt ist der Kunststoffbehälter umfassend mindestens zwei Kompartimentelemente ein Zellkulturgefäß, das aus mindestens einer Zellkulturschale ausgebaut ist.

In einer bevorzugten Ausführungsform gelten die Aussagen, die im Zusammenhang mit dem erfindungsgemäßen Verfahren getroffen wurden, mutatis mutandis auch für den nach dem erfindungsgemäßen Verfahren herstellbaren, bevorzugt hergestellten, Kunststoffbehälter als solchen.

Im Zusammenhang mit der vorliegenden Erfindung wird unter einer "Seitenwand" eine einen offenen oder geschlossenen Hohlraum umschließende Wand eines Kompartimentelements verstanden. Dabei können die jeweiligen Seitenwände der mindestens zwei Kompartimentelemente die Seitenwand des erfindungsgemäßen Kunststoffbehälters bilden. Die Seitenwand eines Kompartimentelements weist dabei eine distale erste und proximale zweite Außenkante auf, wobei die beiden Außenkanten jeweils in einer Ebene liegen, die parallel zur vom Plattenelement gebildeten Ebene verläuft. Weisen das erste oder das zweite Kompartimentelement keine Seitenwand auf, handelt es sich demnach bei dem ersten oder zweiten Kompartimentelement um ein Plattenelement, so entsprechen die Außenkanten dieses ersten oder zweiten Plattenelements der distalen ersten und proximalen zweiten Außenkante einer Seitenwand.

Unter dem Begriff "Kompartimentelement" wird im Zusammenhang mit der vorliegenden Erfindung eine einzelne Baueinheit des zumindest teilweise aus einem thermoplastischen Kunststoff aufgebauten Behälters verstanden. Das Kompartimentelement kann entweder ein Plattenelement sein oder ein Plattenelement und eine um den Außenumfang des Plattenelements angeordnete Seitenwand umfassen. Ist das erste Kompartimentelement ein Plattenelement, so umfasst zumindest das zweite Kompartimentelement ein Plattenelement und eine um den Außenumfang des Plattenelements angeordnete Seitenwand.

Im Zusammenhang mit der vorliegenden Erfindung wird unter "Plattenelement" derjenige Teil eines Kompartimentelements verstanden, der entweder selbst eine distale erste und proximale zweite Außenkante aufweist oder um seinen Außenumfang eine Seitenwand aufweist, welche jeweils eine parallel zum Plattenelement angeordnete distale erste und proximale zweite Außenkante umfasst.

Im Zusammenhang mit der vorliegenden Erfindung wird als "distale" Außenkante die von der vom Plattenelement gebildeten Ebene weiter entfernt liegende Außenkante der um den Außenumfang des Plattenelements angeordneten Seitenwand bezeichnet. Entsprechend wird unter der "proximalen" Außenkante die zur vom Plattenelement gebildeten Ebene näherliegende Außenkante der um den Außenumfang des Plattenelements angeordneten Seitenwand verstanden. Liegen die erste und die zweite Außenkante gleich weit von der vom Plattenelement gebildeten Ebene entfernt, so handelt es sich erfindungsgemäß bei der "distalen" Außenkante um die von der vom Plattenelement des nächsten Kompartimentelements gebildeten Ebene weiter entfernt liegenden Außenkante des Plattenelements selbst oder sofern vorhanden, der um den Außenumfang des Plattenelements angeordneten Seitenwand.

Mit dem Begriff "Verbindungszone" wird erfindungsgemäß der Bereich eines Kompartimentelements bezeichnet, in dem die Verbindung mindestens zweier Kompartimentelemente erfolgt. Erfindungsgemäß besteht die Verbindungszone aus einem thermoplastischen Material, so dass in diesem Bereich eine Plastifizierung des Kompartimentelements durch Kontaktieren mit der mindestens einen temperaturregulierten Düse erfolgt. Die Verbindungszonen eines Kompartimentelements umfassen dabei eine aus thermoplastischem Material bestehende distale erste und eine aus thermoplastischem Material bestehende proximale zweite Außenkante. Typischerweise besitzt jedes Kompartimentelement demnach zwei Verbindungszonen. Handelt es sich bei dem ersten oder zweiten Kompartimentelement jedoch um ein Plattenelement, so kann dieses auch lediglich eine Verbindungzone umfassen.

Unter dem Begriff "und/oder" wird im Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: A oder B oder C, oder (A und B) oder (A und C) oder (B und C) oder (A und B und C).

Im Zusammenhang mit der vorliegenden Erfindung wird unter dem Begriff "umfassend" verstanden, dass zusätzlich zu den von dem Begriff explizit erfassten Elementen noch weitere, nicht explizit genannte Elemente hinzutreten können. Im Zusammenhang mit der vorliegenden Erfindung wird unter diesem Begriff auch verstanden, dass alleine die explizit genannten Elemente erfasst werden und keine weiteren Elemente vorliegen. In dieser besonderen Ausführungsform ist die Bedeutung des Begriffs "umfassend" gleichbedeutend mit dem Begriff "bestehend aus". Darüber hinaus erfasst der Begriff "umfassend" auch Gesamtheiten, die neben den explizit genannten auch weitere nicht genannte Elemente enthalten, die jedoch von funktionell und qualitativ untergeordneter Natur sind. In dieser Ausführungsform ist der Begriff "umfassend" gleichbedeutend mit dem Begriff "im Wesentlichen bestehend aus".

Weitere vorteilhafte Ausgestaltungen der Erfindung ergeben sich aus den Unteransprüchen.

Die vorliegende Erfindung wird im Folgenden anhand der Figuren und dem Ausführungsbeispiel näher erläutert.

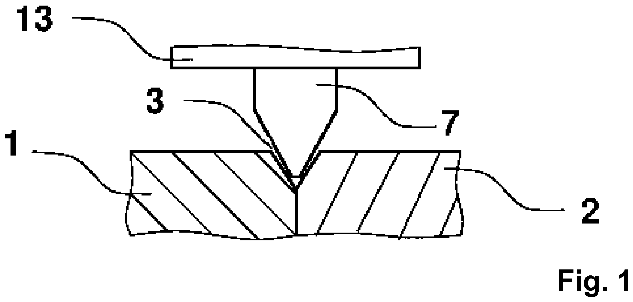

Dabei zeigt **Figur 1** das Eintauchen der temperaturregulierte Düse 7 in die zwischen zwei Kompartimentelementen 1 und 2 liegende Verbindungszone 3. Dabei werden die beiden Kompartimentelemente 1 und 2 in Bereich der Verbindungszone 3 plastifiziert.

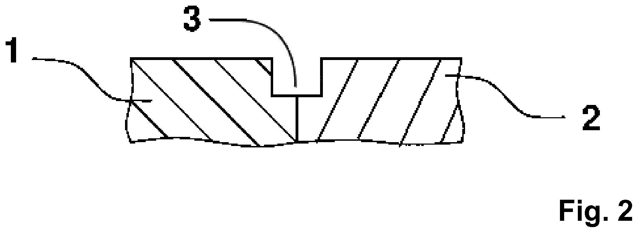

Eine alternative Geometrie der Gestaltung der Verbindungszone 3 ist in **Figur 2** gezeigt. Dabei wird im Bereich der Verbindungszone 3 zwischen den Kompartimentelementen 1 und 2 eine Nut gebildet. Vorteil einer solchen Nut ist, dass plastifizierter Schweißzusatz freier zwischen die plastifizierten Kompartimentelemente 1 und 2 eingebracht werden kann, da im Bereich direkt vor dem Düsenausgang ein Hohlraum entsteht. In diesen Hohlraum kann der Schweißzusatz dosiert werden.

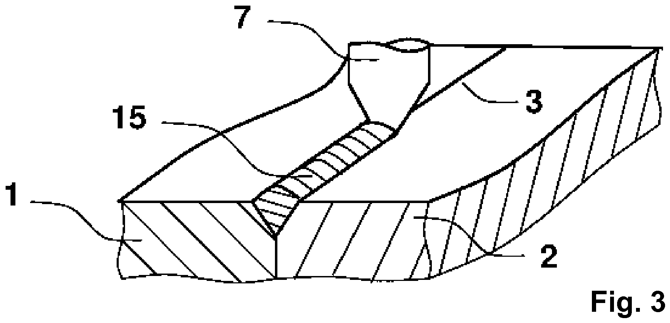

Die Gestaltung der Schweißnaht ist in **Figur 3** gezeigt. Während die temperaturregulierte Düse 7 die beiden Kompartimentelemente 1 und 2 entlang der Verbindungszone 3 plastifiziert, wird gleichzeitig plastifizierter Schweißzusatz 8 in die plastifizierte Verbindungszone 3 appliziert. Dadurch kommt es zum Verschmelzen der im Bereich der Verbindungszone 3 durch die temperaturregulierte Düse 7 plastifizierten Kompartimentelemente 1 und 2 mit dem Schweißzusatz 8, so dass sich nach Abkühlen eine feste Schweißnaht 15 bildet.

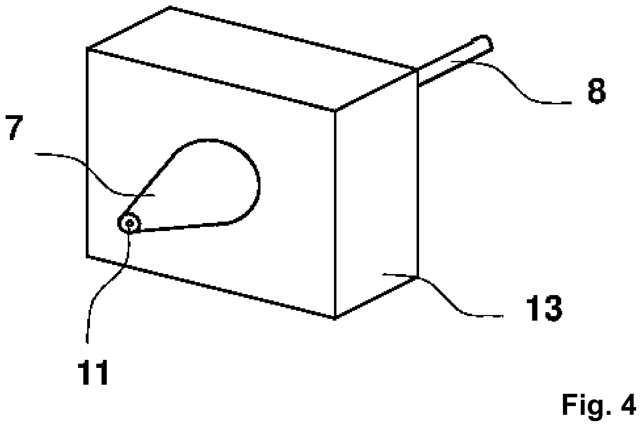

**Figur 4** zeigt den Aufbau der temperaturregulierten Düse 7. Die Düse verjüngt sich vorne zum Düsenausgang 11 hin, um gezielt die Verbindungszone 3 zwischen zwei Kompartimentelementen zu erwärmen und dort den Schweißzusatz 8 zu platzieren.

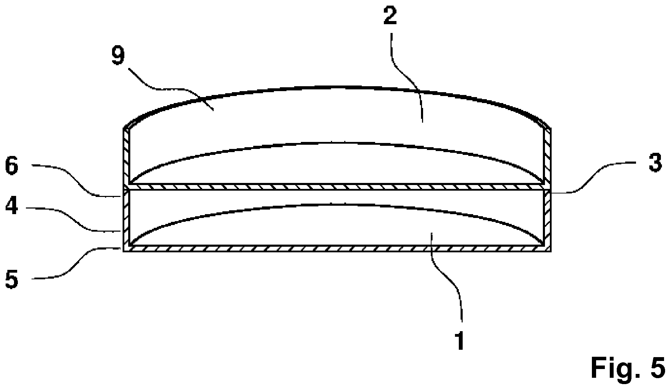

**Figur 5** zeigt einen mit dem Verfahren gemäß der vorliegenden Erfindung hergestellten aus zwei Kompartimentelementen 1 und 2 bestehenden Kunststoffbehälter 9. Dargestellt ist Kompartimentelement 1, umfassend eine distale erste Außenkante 6 und eine proximale zweite Außenkante 5 der Seitenwand 4, das in Bereich der Verbindungszone 3 mit einem zweiten Kompartimentelement 2 verschweißt wurde.

### Beispiel:

Eine zum Verschweißen von mindestens zwei Kompartimentelementen gemäß dem erfindungsgemäßen Verfahren geeignete temperaturregulierte Düse 7 ist in Figur 4 abgebildet. Die Düse 7 ist in einen Heizblock 13 eingeschraubt. Dieser Heizblock 13 wird z. B. über eine Heizpatrone und einen Fühler auf eine gewünschte Temperatur geregelt. Durch thermische Leitung wird die Temperatur des Heizblocks 13 auf die Düse 7 übertragen, so dass diese temperaturreguliert betrieben wird. Der Schweißzusatz 8 wird von der Rückseite der Düse zugeführt. Hierbei ist es möglich, den Schweißzusatz 8 in Form eines Schweißdrahts zuzuführen. Der Vorteil den Schweißzusatz 8 als Schweißdraht zuzuführen, liegt darin, dass Schweißdraht mit einem Durchmesser von 1,75 mm oder 2,85 mm gewöhnlich auch in 3D-Druckern verwendet wird und damit standardmäßig zur Verfügung steht. Schweißzusatz in Form von Schweißdraht kann durch einen Motor in Kombination mit einer Rändelschraube gefördert oder auch zurückgezogen werden. Dadurch ist eine genaue Dosierung möglich. Alternativ ist es aber beispielsweise auch die Verwendung von Granulat möglich. Bei der Verwendung von Granulat ist die Bevorratung und Verteilung des Schweißzusatzes 8 zu den Düsen 7 flexibler. Dies ist insbesondere von Bedeutung, wenn mehrere Schweißungen parallel durchgeführt werden, wenn also mehrere Düsen gleichzeitig mit Schweißzusatz versorgt werden müssen. In dem hier beschriebenen Verfahrensbeispiel wird eine Düse 7 mit einer vorderen Öffnung 11 mit einem Durchmesser von 0,5 mm bis 1 mm verwendet. Dies erlaubt eine Breite der Schweißnaht 15 von ca. 2 mm. Andere Durchmesser der Öffnung 11 ändern neben der Breite der Schweißnaht 15 auch die Festigkeit der Verschweißung.

Im Falle eines in Figur 5 dargestellten zylindrischen Kunststoffbehälters kann die temperaturregulierte Düse 7 in die Verbindungszone 3 gefahren werden, um diese zu plastifizieren. Gleichzeitig dosiert die temperaturregulierte Düse 7 den Schweißzusatz 8 um den Umfang des zylindrischen Kunststoffbehälters 9 zwischen die Kompartimentelemente 1 und 2. In einer einfachen Ausführungsform der vorliegenden Erfindung rotiert der zylindrische Kunststoffbehälter 9 um seine eigene Achse, während die Düse 7 ortsfest bleibt.

Bei Kunststoffbehältern, die keine zylindrische Form besitzen, muss die temperaturregulierte Düse 7 entlang der zwischen zwei Kompartimentelementen gebildeten Verbindungszone 3 geführt werden. Dies kann z. B. mit einem 3-Achsen-System, ähnlichen einem 3D-Drucker oder einer Fräsmaschine, umgesetzt werden. Auch die Verwendung von Gelenkarm- oder Hexapod-Systemen ist denkbar.

## Patentansprüche

1. Ein Verfahren zur Herstellung eines zumindest teilweise aus einem thermoplastischen Kunststoff aufgebauten Behälters, umfassend mindestens ein erstes Kompartimentelement und mindestens ein mit dem ersten in einem Verbindungsbereich thermoplastisch verschweißtes zweites Kompartimentelement, wobei das Verfahren folgende Verfahrensschritte umfasst:
a) Bereitstellen mindestens eines ersten und eines zweiten Kompartimentelements (1, 2), welche jeweils ein Plattenelement mit mindestens einer die Außenkanten des Kompartimentelements umfassenden Verbindungszone (3) aus einem thermoplastischen Material aufweisen, wobei zumindest das zweite Kompartimentelement eine um den Außenumfang des Plattenelements angeordnete Seitenwand (4) mit jeweils einer distalen ersten und proximalen zweiten Außenkante (6, 5) umfasst,
b) Aneinanderfügen der mindestens zwei Kompartimentelemente (1, 2), so dass sich die Verbindungszonen (3) aus thermoplastischem Kunststoff kontaktieren und eine Außenkante des ersten Kompartimentelements vollumfänglich an die erste Außenkante des zweiten Kompartimentelements stößt und ein vollumfänglicher Stumpfstoß zwischen zwei Kompartimentelementen (1, 2) gebildet wird,
c) Verschweißen der mindestens zwei Kompartimentelemente (1, 2) mittels mindestens einer temperaturregulierten Düse (7), wobei die mindestens eine Düse (7) den Stumpfstoß kontaktierend um den Umfang des Stumpfstoßes geführt wird oder der Stumpfstoß die mindestens eine Düse (7) kontaktierend an der mindestens einen Düse (7) entlanggeführt wird, wobei dabei die jeweilige erste und zweite Außenkante (6, 5) der Kompartimentelemente plastifiziert wird und gleichzeitig plastifizierter Schweißzusatz (8) auf den Stumpfstoß übertragen wird, so dass eine Schweißnaht (15) zwischen den beiden Kompartimentelementen (1, 2) gebildet wird, und
d) Erhalt eines Kunststoffbehälters (9), wobei die mindestens zwei Kompartimentelemente (1, 2) miteinander verschweißt sind.

2. Das Verfahren nach Anspruch 1, wobei das erste Kompartimentelement eine um den Außenumfang des Plattenelements angeordnete Seitenwand (4) mit jeweils einer parallel zum Plattenelement angeordneten distalen ersten und proximalen zweiten Außenkante (6, 5) umfasst.

3. Das Verfahren nach einem der Ansprüche 1 und 2, wobei in Verfahrensschritt a) mindestens ein weiteres Kompartimentelement bereitgestellt wird, in Verfahrensschritt b) das weitere Kompartimentelement mit seiner ersten Außenkante (6) vollumfänglich an die zweite Außenkante (5) des zweiten oder eines gegebenenfalls vorliegenden weiteren Kompartimentelements angefügt wird und in Verfahrensschritt c) der zwischen den aneinandergefügten Kompartimentelementen entstandene Stumpfstoß verschweißt wird.

4. Das Verfahren nach einem der vorstehenden Ansprüche, wobei jedes weitere Kompartimentelement ein Plattenelement und eine um den Außenumfang des Plattenelements angeordnete Seitenwand (4) mit jeweils einer parallel zum Plattenelement angeordneten distalen ersten und proximalen zweiten Außenkante (6, 5) umfasst.

5. Das Verfahren nach einem der vorstehenden Ansprüche, wobei in Verfahrensschritt b) die Kompartimentelemente gegeneinander drehsicher aneinandergefügt werden.

6. Das Verfahren nach einem der vorstehenden Ansprüche, wobei mindestens ein Kompartimentelement und der Schweißzusatz (8) aus den gleichen Materialien bestehen.

7. Das Verfahren nach einem der vorstehenden Ansprüche, wobei die mindestens eine Düse (7) den Schweißzusatz (8) plastifiziert.

8. Das Verfahren nach einem der vorstehenden Ansprüche, wobei mindestens eines der mindestens zwei Kompartimentelemente (1, 2) aus einem Material besteht, umfassend ein oder mehrere Thermoplasten ausgewählt aus der Gruppe bestehend aus Polystyrol, Polypropylen, Polycarbonat, Polyethylenterephthalat und Polyethylen.

9. Das Verfahren nach einem der vorstehenden Ansprüche, wobei das Material des Schweißzusatzes (8) ein oder mehrere Thermoplasten ausgewählt aus der Gruppe bestehend aus Polystyrol, Polypropylen, Polycarbonat, Polyethylenterephthalat und Polyethylen ist.

10. Das Verfahren nach einem der vorstehenden Ansprüche, wobei der Schweißzusatz (8) in Form von Granulat der mindestens einen Düse (7) zugeführt wird.

11. Das Verfahren nach einem der Ansprüche 1 bis 9, wobei der Schweißzusatz (8) in Form eines kontinuierlichen Strangs der mindestens einen Düse (7) zugeführt wird.

12. Das Verfahren nach einem der vorstehenden Ansprüche, wobei in einem weiteren Verfahrensschritt b') die Kompartimentelemente im Bereich des Stumpfstoßes vorgewärmt werden.

13. Das Verfahren nach Anspruch 12, wobei in Verfahrensschritt b') die Kompartimentelemente im Bereich des Stumpfstoßes durch Heißluft, Infrarot- und/oder Mikrowellenstrahlung vorgewärmt werden.

14. Das Verfahren nach einem der vorstehenden Ansprüche, wobei in einem weiteren Verfahrensschritt c') die Schweißnaht (15) erwärmt wird.

15. Das Verfahren nach Anspruch 14, wobei in Verfahrensschritt c') die Schweißnaht (15) durch Heißluft, Infrarot- und/oder Mikrowellenstrahlung erwärmt wird.

16. Das Verfahren nach einem der vorstehenden Ansprüche, wobei durch das aufeinanderstoßen der Kompartimentelemente in Verfahrensschritt b) mindestens eine Vertiefung im Bereich des Stumpfstoßes zwischen den Kompartimentelementen gebildet wird.

17. Das Verfahren nach Anspruch 16, wobei die mindestens eine temperaturregulierte Düse (7) in Verfahrensschritt c) vollständig oder partiell in die mindestens eine Vertiefung eingeführt wird.

18. Das Verfahren nach einem der vorstehenden Ansprüche, wobei die Kompartimentelemente für die Kultivierung und/oder Untersuchung von Zellen geeignet sind.

19. Das Verfahren nach Anspruch 18, wobei mindestens ein Kompartimentelement eine Zellkulturschale ist.

20. Das Verfahren nach einem der Ansprüche 18 und 19, wobei das mindestens eine Kompartimentelement einen verbindenden Kanal als Versorgungskanal für Zellkulturmedium aufweist.

21. Das Verfahren nach Anspruch 20, wobei mindestens ein Kompartimentelement einen Entsorgungskanal für verbrauchtes Zellkulturmedium aufweist.

22. Kunststoffbehälter (9) umfassend mindestens zwei Kompartimentelemente (1, 2) herstellbar nach einem der vorstehenden Verfahren.

## Claims

1. A method for producing a container which is at least partially constructed from a thermoplastic material, comprising at least one first compartment element and at least one second compartment element which is thermoplastically welded to the first in a joining region, the method comprising the following method steps:
a) providing of at least one first and one second compartment element (1, 2), each having a plate element with at least one joining zone (3) of a thermoplastic material, comprising the outer edges of the compartment element, at least the second compartment element comprising a side wall (4) arranged around the outer circumference of the plate element and each having a distal first and a proximal second outer edge (6, 5),
b) joining together of the at least two compartment elements (1, 2) such that the joining zones (3) of thermoplastic material contact each other and one outer edge of the first compartment element fully abuts the first outer edge of the second compartment element and a full butt joint is formed between the two compartment elements (1, 2),
c) welding of the at least two compartment elements (1, 2) by means of at least one temperature-regulated nozzle (7), wherein the at least one nozzle (7) is guided around the circumference of the butt joint while in contact to the butt joint, or the butt joint is guided along the at least one nozzle (7) while in contact to the at least one nozzle (7), wherein the respective first and second outer edges (6, 5) of the compartment elements are plasticized and simultaneously plasticized welding filler (8) is transferred to the butt joint so that a weld seam (15) is formed between the two compartment elements (1, 2), and
d) obtaining a plastic container (9), wherein the at least two compartment elements (1, 2) are welded together.

2. The method according to claim 1, wherein the first compartment element comprises a side wall (4) arranged around the outer circumference of the plate element, each having a distal first and proximal second outer edge (6, 5) arranged parallel to the plate element.

3. The method according to one of claims 1 and 2, wherein in method step a) at least one further compartment element is provided, in method step b) the further compartment element is fully joined with its first outer edge (6) to the second outer edge (5) of the second compartment element or any further compartment element present, and in method step c) the butt joint formed between the joined compartment elements is welded.

4. The method according to any one of the preceding claims, wherein each further compartment element comprises a plate element and a side wall (4) arranged around the outer circumference of the plate element, each side wall having a distal first and proximal second outer edge (6, 5) arranged parallel to the plate element.

5. The method according to any one of the preceding claims, wherein in method step b) the compartment elements are joined together in a rotationally secure manner with respect to each other.

6. The method according to any one of the preceding claims, wherein at least one compartment element and the welding filler (8) consist of the same materials.

7. The method according to any one of the preceding claims, wherein the at least one nozzle (7) plasticizes the welding filler (8).

8. The method according to any one of the preceding claims, wherein at least one of the at least two compartment elements (1, 2) consist of a material comprising one or more thermoplastics selected from the group consisting of polystyrene, polypropylene, polycarbonate, polyethylene terephthalate and polyethylene.

9. The method according to any one of the preceding claims, wherein the material of the welding filler (8) is one or more thermoplastics selected from the group consisting of polystyrene, polypropylene, polycarbonate, polyethylene terephthalate and polyethylene.

10. The method according to any one of the preceding claims, wherein the welding filler (8) is supplied in the form of granules to the at least one nozzle (7).

11. The method according to any one of claims 1 to 9, wherein the welding filler (8) is supplied in the form of a continuous strand to the at least one nozzle (7).

12. The method according to one of the preceding claims, wherein in a further method step b') the compartment elements in the region of the butt joint are preheated.

13. The method according to claim 12, wherein in method step b') the compartment elements in the region of the butt joint are preheated by hot air, infrared and/or microwave radiation.

14. The method according to one of the preceding claims, wherein in a further method step c') the weld seam (15) is heated.

15. The method according to claim 14, wherein in method step c') the weld seam (15) is heated by hot air, infrared and/or microwave radiation.

16. The method according to any one of the preceding claims, wherein at least one recess is formed in the region of the butt joint between the compartment elements by the abutting of the compartment elements in method step b).

17. The method according to claim 16, wherein the at least one temperature-regulated nozzle (7) in method step c) is fully or partially immerged into the at least one recess.

18. The method according to any one of the preceding claims, wherein the compartment elements are suitable for the cultivation and/or examination of cells.

19. The method according to claim 18, wherein at least one compartment element is a cell culture dish.

20. The method of any one of claims 18 and 19, wherein the at least one compartment element has a connecting channel as a supply channel for cell culture medium.

21. The method according to claim 20, wherein at least one compartment element has a disposal channel for used cell culture medium.

22. Plastic container (9) comprising at least two compartment elements (1, 2) producible according to one of the preceding methods.

## Revendications

1. Procédé de fabrication d'un récipient constitué au moins partiellement d'un matériau thermoplastique, comprenant au moins un premier élément de compartiment et au moins un deuxième élément de compartiment soudé de manière thermoplastique au premier dans une zone de liaison, le procédé comprenant les étapes de procédé suivantes
a) Fournir au moins un premier et un second élément de compartiment (1, 2), chacun ayant un élément de plaque avec au moins une zone de connexion (3) faite d'un matériau thermoplastique et comprenant les bords extérieurs de l'élément de compartiment, au moins le second élément de compartiment comprenant une paroi latérale (4) disposée autour de la circonférence extérieure de l'élément de plaque et ayant chacun un premier bord extérieur distal et un second bord extérieur proximal (6, 5),
b) Assembler les au moins deux éléments de compartiment (1, 2) de manière à ce que les zones de connexion (3) en matériau thermoplastique soient en contact l'une avec l'autre et qu'un bord extérieur du premier élément de compartiment vienne entièrement en butée contre le premier bord extérieur du second élément de compartiment et qu'un joint bout à bout sur toute la circonférence soit formé entre deux éléments de compartiment (1, 2),
c) Souder les au moins deux éléments de compartiment (1, 2) au moyen d'au moins une buse (7) régulée en température, dans lequel la au moins une buse (7) est guidée autour de la circonférence du joint bout à bout en contact avec le joint bout à bout ou le joint bout à bout est guidé le long de la au moins une buse (7) en contact avec la au moins une buse (7), dans lequel les premier et second bords extérieurs respectifs (6, 5) des éléments de compartiment sont plastifiés et, en même temps, un apport de soudage plastifiée (8) est transférée au joint bout à bout de sorte qu'un cordon de soudure (15) est formé entre les deux éléments de compartiment (1, 2), et
d) Obtenir un récipient en plastique (9), les au moins deux éléments de compartiment (1, 2) étant soudés ensemble.

2. Le procédé selon la revendication 1, dans lequel le premier élément de compartiment comprend une paroi latérale (4) disposée autour de la circonférence extérieure de l'élément de plaque, chacune ayant un premier bord extérieur distal et un second bord extérieur proximal (6, 5) disposés parallèlement à l'élément de plaque.

3. Le procédé selon l'une des revendications 1 et 2, dans lequel, à l'étape a) du procédé, au moins un autre élément de compartiment est fourni, à l'étape b) du procédé, l'autre élément de compartiment est entièrement relié par son premier bord extérieur (6) au deuxième bord extérieur (5) du deuxième élément de compartiment ou d'un autre élément de compartiment éventuellement présent, et à l'étape c) du procédé, le joint bout à bout formé entre les éléments de compartiment reliés est soudé.

4. Le procédé selon l'une quelconque des revendications précédentes, dans lequel chaque autre élément de compartiment comprend un élément de plaque et une paroi latérale (4) disposée autour de la circonférence extérieure de l'élément de plaque, chaque paroi latérale ayant un premier bord extérieur distal et un second bord extérieur proximal (6, 5) disposée parallèlement à l'élément de plaque.

5. Le procédé selon l'une quelconque des revendications précédentes, dans lequel, à l'étape b) du procédé, les éléments de compartiment sont reliés les uns aux autres de manière fixe en rotation les uns par rapport aux autres.

6. Le procédé selon l'une quelconque des revendications précédentes, dans lequel au moins un élément de compartiment et l'apport de soudure (8) sont constitués des mêmes matériaux.

7. Le procédé selon l'une quelconque des revendications précédentes, dans lequel la au moins une buse (7) plastifie l'apport de soudure (8).

8. Le procédé selon l'une quelconque des revendications précédentes, dans lequel au moins l'un des au moins deux éléments de compartiment (1, 2) est réalisé en un matériau comprenant un ou plusieurs thermoplastiques choisis dans le groupe constitué par le polystyrène, le polypropylène, le polycarbonate, le polyéthylène téréphtalate et le polyéthylène.

9. Le procédé selon l'une quelconque des revendications précédentes, dans lequel le matériau de l'apport de soudage (8) est un ou plusieurs thermoplastiques choisis dans le groupe constitué par le polystyrène, le polypropylène, le polycarbonate, le polyéthylène téréphtalate et le polyéthylène.

10. Le procédé selon l'une quelconque des revendications précédentes, dans lequel l'apport de soudure (8) est amenée sous forme de granulés à la au moins une buse (7).

11. Le procédé selon l'une quelconque des revendications 1 à 9, dans lequel l'apport de soudure (8) est amenée sous la forme d'un cordon continu à la au moins une buse (7).

12. Le procédé selon l'une quelconque des revendications précédentes, dans lequel, dans une autre étape b') du procédé, les éléments de compartiment sont préchauffés dans la région du joint bout à bout.

13. Le procédé selon la revendication 12, dans lequel, à l'étape b') du procédé, les éléments de compartiment sont préchauffés dans la région du joint bout à bout par de l'air chaud, un rayonnement infrarouge et/ou un rayonnement micro-ondes.

14. Le procédé selon l'une quelconque des revendications précédentes, dans lequel, dans une autre étape du procédé c'), le cordon de soudure (15) est chauffé.

15. Le procédé selon la revendication 14, dans lequel, à l'étape c') du procédé, le cordon de soudure (15) est chauffé par de l'air chaud, un rayonnement infrarouge et/ou un rayonnement micro-ondes.

16. Le procédé selon l'une quelconque des revendications précédentes, dans lequel au moins un évidement est formé dans la région du joint bout à bout entre les éléments de compartiment par l'aboutement des éléments de compartiment à l'étape b) du procédé.

17. Le procédé selon la revendication 16, dans lequel l'au moins une buse régulée en température (7) est totalement ou partiellement insérée dans l'au moins un évidement lors de l'étape c) du procédé.

18. Le procédé selon l'une quelconque des revendications précédentes, dans lequel les éléments de compartiment sont adaptés à la culture et/ou à l'examen de cellules.

19. Le procédé selon la revendication 18, dans lequel au moins un élément de compartiment est une boîte de culture cellulaire.

20. Le procédé de l'une quelconque des revendications 18 et 19, dans lequel le au moins un élément de compartiment a un canal de connexion comme canal d'alimentation pour le milieu de culture cellulaire.

21. Le procédé selon la revendication 20, dans lequel le au moins un élément de compartiment a un canal d'évacuation pour le milieu de culture cellulaire usagé.

22. Récipient en plastique (9) comprenant au moins deux éléments de compartiment (1, 2) pouvant être produits par l'un quelconque des procédés précédents.
